(19) Europäisches Patentamt
European Patent Office
Office européen des brevets

(11) Publication number: **0 253 489 B1**

(12) # EUROPEAN PATENT SPECIFICATION

(45) Date of publication of patent specification: **10.03.93** (51) Int. Cl.$^5$: **A61K 7/08**

(21) Application number: **87305075.1**

(22) Date of filing: **09.06.87**

(54) **Antidandruff shampoo composition having improved suspension properties.**

(30) Priority: **16.06.86 US 874542**

(43) Date of publication of application:
**20.01.88 Bulletin 88/03**

(45) Publication of the grant of the patent:
**10.03.93 Bulletin 93/10**

(84) Designated Contracting States:
**AT BE CH DE ES FR GB IT LI LU NL SE**

(56) References cited:
**EP-A- 0 117 135**
**EP-A- 0 200 305**
**EP-A- 0 210 774**
**GB-A- 2 015 562**

(73) Proprietor: **HELENE CURTIS, INC.**
**325 North Wells Street**
**Chicago Illinois 60610(US)**

(72) Inventor: **LaPetina, Donna M.**
**2 S 030 Bristol Ln**
**Warrenville Illinois 60555(US)**
Inventor: **Patel, Chaitanya**
**1118-1A Prescott**
**Roselle Illinois 60172(US)**

(74) Representative: **Wain, Christopher Paul et al**
**A.A. THORNTON & CO. Northumberland**
**House 303-306 High Holborn**
**London WC1V 7LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

The present invention relates to an antidandruff hair shampoo composition including a suspension composition capable of achieving a substantial and unexpected reduction in separation of solid, particulate matter, particularly a solid antidandruff agent, while maintaining acceptable shampoo viscosities and foaming levels. More particularly, the present invention is directed to an antidandruff shampoo composition containing discrete suspended, solid antidandruff agent particles, such as zinc pyrithione or sulfur, and to a suspension composition for the antidandruff shampoo including a suspending alkanolamide and/or a wax ester in conjunction with an ethylene-maleic anhydride resin or a polyacrylic acid resin. The suspension composition, present at a low level of 3% maximum, imparts improved antidandruff agent suspension over a wide range of viscosities.

The incorporation of antidandruff agents into anionic surfactant-based hair shampoos is well known. The individual antidandruff agents must not only relieve the flaking and itching symptoms of dandruff, but also be substantive to the skin and hair in order to extend the antidandruff agent's efficacy from one treatment to the next. These properties are most often found in compounds not soluble in aqueous media, and this inherent insolubility of the antidandruff agents makes formulation of a stable, aqueous, anionic surfactant-based antidandruff shampoo a difficult problem.

In order to incorporate such effective, sparsely soluble antidandruff agents as zinc pyrithione or sulfur into aqueous anionic surfactant-based hair shampoos, one or more suspending agents are required to keep the antidandruff agent homogeneously dispersed throughout the aqueous solution. Failure to adequately suspend the antidandruff agent leads to eventual shampoo separation as the antidandruff agent settles to the bottom of the container, and results in poor dandruff control and consumer complaints. Early antidandruff shampoo compositions used bentonite clay as the suspending agent, however, the trend has been to avoid bentonite since it gives the shampoo a dirty appearance and dries the hair due to the substantial oil absorption characteristic of bentonite. As a result, there has been a continuous search for suitable suspending agents capable of effectively dispersing antidandruff agents such as zinc pyrithione or sulfur.

In general, compositions containing insoluble particulate matter require a suspending agent to assist in dispersing the particulate matter evenly throughout the composition. Depending upon the ultimate use of the composition, the suspending agent may be any one of a number of inorganic minerals or synthetic or natural polymers or gums. Among the most often used suspension agents are colloidal aluminum oxide, modified magnesium aluminum silicate, xanthan gum, fumed silica, algin products, polyacrylic acid, sodium carboxymethylcellulose, hydroxypropylcellulose, synthetic sodium magnesium silicate, colloidal attapulgite clay, lignins and alkanolamides. In most compositions, the addition of a suspending agent at a great enough percentage to adequately suspend the particles in solution leads to an increase in viscosity.

Common hair shampoos are known to contain an effective low-to-non-irritating amount of an anionic surfactant, usually an alkyl sulfate, as a principal cleansing agent. Hair shampoos also include other components to improve product efficacy, stability and consumer acceptance. Therefore, any antidandruff agent and additional suspending agents added to a basic hair shampoo is expected to add antidandruff properties to the shampoo without detracting from the cleaning efficiency and aesthetic appeal of the shampoo. Unfortunately, the antidandruff agents and necessary suspending agents often adversely affect the foaming characteristics of the shampoo composition. Consumers perceive a substantial benefit in copious suds formation, since they incorrectly equate copious foam with excellent cleaning, and poor sudsing with inferior shampoos. The decreased foaming capability of the antidandruff shampoos may be overcome by increasing the surfactant level of the shampoo, however, this option usually is avoided due to economic factors and oil extraction from the hair by detergent overformation.

Consumer acceptance of antidandruff hair shampoos also is influenced by the viscosity of the shampoo. The ideal shampoo should be thick enough to appear concentrated and not to run out of the container or hand too easily during application, and be thin enough for easy dispensing from the container, ease of application to the hair and even distribution over the scalp. These characteristics usually are found in a viscosity range from 2000 cps to 8000 cps. Some antidandruff suspending agent compositions tend to increase the viscosity of the shampoo outside consumer acceptable limits.

U.S. Patent No. 4,470,982 (Winkler), discloses a suspending agent composition for antidandruff agents including 4% to 6% of either the ethylene glycol esters of fatty acids having from 16 to 22 carbon atoms; alkanolamides of fatty acids having from 16 to 22 carbon atoms; or alkyl ($C_{16}$-$C_{22}$) dimenthyl amine oxides to produce antidandruff shampoo compositions of good viscosity and acceptably low separation. In accordance with the Winkler patent, the levels of suspending agent, surfactant and alkanolamide are critical to the utility of the shampoo compositions.

It is also known from GB-A-2015562 to provide fatty acid alkanolamides in a shampoo composition. However these are detergent liquids and comparatively soluble in water.

It is an object of the invention to provide an improved antidandruff shampoo of the kind based on anionic surfactants and containing a particulate antidandruff agent, the shampoo having reduced shampoo separation and acceptable levels of viscosity and foaming.

We have now found that the addition of a small amount (0.1 to 1% by weight) of a polymer containing a plurality of neutralised carboxyl groups to an anionic surfactant - based antidandruff shampoo significantly and unexpectedly improves the particulate suspension properties of water-insoluble solid alkanolamide and/or wax esters suspending agents. The combination of the neutralised carboxyl group-containing polymer with the alkanolamide and/or wax ester provides an antidandruff shampoo composition which is unexpectedly resistant to composition separation and which has good viscosity and foaming properties.

According to one aspect of the present invention, there is provided an antidandruff shampoo comprising, by weight, from 5 to 20% of an anionic surfactant and from 0.2 to 5% of a particulate antidandruff agent, characterised in that it comprises, by weight:

from 1 to 3% of a water-insoluble suspending agent which is solid at room temperature, which has a solubility in water of less than 1%, and which is a solid alkanolamide or a solid wax ester or a mixture thereof;

from 0.1 to 1% of a resin containing a plurality of carboxyl groups neutralised to a degree sufficient to achieve a composition pH of 5 to 6.5; and

a liquid carrier.

According to another aspect of the present invention, there is provided a suspending composition for suspending particulate matter in an anionic surfactant-based liquid carrier composition, characterised in that it comprises, by weight, based on the final composition:

from 1 to 3% of a water-insoluble suspending agent which is solid at room temperature, which has a solubility in water of less than 1%, and which is a solid alkanolamide or a solid wax ester or a mixture thereof; and

from 0.1 to 1% of a resin containing a plurality of carboxyl groups neutralised to a degree sufficient to achieve a composition pH of 5 to 6.5.

For the better understanding of the invention, reference will be made in the following description to the accompanying drawings, in which:

FIG. 1 is a graph comparing the ability of coconut monoethanolamide and coconut diethanolamide to suspend an antidandruff agent in the hair shampoo composition of Winkler Patent No. 4,470,982;

FIG. 2 is a graph comparing the ability of three suspending agent compositions: EMA alone; a suspending alkanolamide and a wax ester; and a suspending alkanolamide, a wax ester and EMA-to suspend zinc pyrithone in a hair shampoo composition;

FIGS. 3 and 4 show containers used to test the antidandruff hair shampoo compositions for percent separation;

FIG. 5 is a graph comparing the ability of ethylene glycol distearate and stearamide MEA stearate to suspend zinc pyrithione in a hair shampoo composition;

FIG. 6 is a graph comparing the ability of a suspending agent composition of the present invention to suspend sulfur and zinc pyrithione in a hair shampoo composition;

FIG. 7 is a graph comparing the ability of various percentages of EMA to suspend zinc pyrithione in a hair shampoo composition containing a fixed amount of suspending alkanolamide and wax ester; and

FIG. 8 is a graph comparing the suspension capability of the suspending agent composition of the present invention to a leading commercial antidandruff shampoo composition.

The antidandruff hair shampoo compositions of the present invention are liquid anionic surfactant-based shampoos containing an insoluble antidandruff agent homogeneously dispersed and suspended throughout the composition. The antidandruff shampoo composition serves to cleanse the hair and relieve the itching and flaking symptoms of dandruff, while retaining the essential commercial qualities of suitable viscosity and copious foam.

The antidandruff agents of the present invention are any particulate compound capable of relieving the symptoms of dandruff and that are substantive to the hair and scalp to afford residual antidandruff properties between shampoos. Among the many particulate compounds exhibiting antidandruff properties are salicylic acid, elemental sulfur, selenium dioxide, zinc pyrithione, other 1-hydroxy pyridones and the azole antimycotics. Particularly advantageous antidandruff agents useful in the shampoo composition of the present invention are zinc pyrithione and elemental sulfur. Zinc pyrithione is the zinc complex of 2-pyridinethiol-1-oxide, and is available commercially from Olin Corp. under the brand name of ZINC OMADINE. Useful sulfurs include elemental sulfur of sufficient purity and particle size to function as an

antidandruff agent, as well known in the art.

The antidandruff agents are extremely insoluble and, therefore, are present in the antidandruff shampoo composition as discrete solid particles. These particles should be homogeneously dispersed and suspended throughout the shampoo to ensure the consumer of an efficacious dose of antidandruff agent at each shampooing. Without a suspending agent composition, the antidandruff agent may completely separate from the hair shampoo composition resulting in poor dandruff control, and ultimately in consumer dissatisfaction and complaints. Therefore, a suspending agent composition must be incorporated into the basic antidandruff formulation to retard, minimize or eliminate settling of the insoluble antidandruff agent.

The antidandruff agent is present in the shampoo in a topically effective amount, generally between 0.25% and 5% by total weight of the shampoo. These limits are well above the solubility limits of the antidandruff agents in water (e.g., zinc pyrithione is 0.0015% by weight soluble in water), and, therefore, are incorporated into the shampoo formulation as discrete solid particles in suspension.

The ideal suspending agent composition homogeneously disperses the antidandruff particles throughout the composition for an indefinite period of time without affecting the viscosity, foaming, cleaning, or antidandruff properties of the shampoo. Many suspending agents operate on the principle of thickening the liquid to a great enough viscosity to retard the settling of particulate matter, having a diameter of from about 2 to about 7 $\mu$m (microns), to such an extent that the product is stable over its lifetime. However, considering the relatively high percentage of antidandruff agent incorporated into antidandruff shampoos, a suspending agent relying only on thickening must be incorporated in such a high percentage to suspend the antidandruff agent that an unacceptably viscous product results. Shampoos having such a high viscosity are not acceptable to consumers since they are hard to dispense, hard to spread evenly on the hair and scalp, and often do not generate adequate foam.

As will become more apparent hereinafter, the suspending agent compositions of the present invention suspends a particulate antidandruff agent to an unexpectedly high degree, while maintaining viscosities suitable for hair compositions. The suspending agent compositions of the present invention are included in the shampoo in unexpectedly and unusually low levels, simplifying retention of the proper foam characteristics of the shampoo. The suspending agent compositions of the antidandruff shampoos of the present invention include a suspending alkanolamide and a wax ester, alone or in combination, in weight percentages of from 1% to 3% (preferably 1.5 to 2.5%) by total weight of the shampoo. As will be seen, the alkanolamides useful for particle suspension differ from the alkanolamides incorporated into the shampoo to help cleanse the hair. The suspension alkanolamides are water insoluble waxy type solids, such as stearamide MEA stearate, stearamide DEA stearate or stearamide DIBA stearate. As will become apparent, these high melting point waxy alkanolamides (generally at least 30 carbon atoms in length) of low water solubility (less than 1% by weight) suspend particulate matter in anionic surfactant-based shampoo systems to an unexpected degree when introduced in relatively low percentages. In addition to or instead of the suspending alkanolamides, a wax ester may be used as the suspending agent. Such wax esters include, for example, stearyl stearates, myristyl myristate, myristyl stearate, cetyl myristate, cetyl stearate, ethylene glycol monostearate, ethylene glycol distearate, diethylene glycol monostearate, diethylene glycol distearate, propylene glycol monostearate, propylene glycol distearate, and propylene glycol monolaurate; however, any solid, less than 1% by weight water soluble waxy ester will serve to disperse and suspend the particulate antidandruff agent of the antidandruff shampoo compositions of the present invention. Prior to the present invention, it was impossible to provide an antidandruff shampoo having good suspension, viscosity and foaming characteristics with a suspending agent composition at a level of 4% by weight or less, of the shampoo.

It has been found that the wax esters and/or alkanolamides described above will not suspend the particulate antidandruff agent satisfactorily at the levels of 4% by weight or less for extended periods of time. However, in accordance with an important feature of the present invention, the addition of a low percentage, from 0.1% to 1% (preferably 0.3-0.6%) by weight of a resin containing a plurality of neutralized carboxyl groups serves to complement the wax ester and/or suspending alkanolamide to unexpectedly increase the separation stability of the antidandruff shampoo so that the level of alkanolamide and/or wax ester can be reduced to 1% to 3% by weight of the shampoo.

To achieve the full advantage of the present invention, the carboxylic acid-containing resins should be ethylene-maleic anhydride resins and/or polyacrylic acid resins. As supplied, the carboxyl-containing resins are in the acid or anhydride form and are neutralized with a suitable base such that the shampoo composition of the present invention has a pH of from 5 to 6.5. Generally, the degree of neutralization required to achieve a shampoo pH in the range of 5 to 6.5 is 90% to 100% neutralization of the carboxyl groups of the resin. The resins are available in crosslinked or un-crosslinked forms, with the crosslinked resins showing the greatest utility in the shampoo composition of the present invention. The ethylene-maleic

anhydride (EMA) resin or polyacrylic acid resin may be neutralized by any standard base such as the alkali metal hydroxides, ammonium hydroxide, or alkylamines containing one to four carbon atoms, to provide a neutralized, water dispersible, crosslinked resin able to aid, unexpectedly, to suspend insoluble particulate matter.

Neutralized crosslinked resins such as those used in the shampoo composition of the present invention usually lead to very rapid solution viscosity increases, even when used at very low concentrations, normally making the resins unsatisfactory for use in hair care compositions. However, quite unexpectedly, in accordance with the present invention, the combination of one or more resins having a plurality of carboxyl groups with a wax ester and/or alkanolamide does not lead to unsuitably high viscosity levels. To achieve the full advantage of the present invention, the use of low levels of a neutralized EMA or polyacrylic acid resin with low levels of a suspending alkanolamide and/or wax ester synergistically permits the total percentage of the suspension system in the composition to remain low, at a level of 4% by weight or less, thus maintaining acceptable viscosity levels while reducing the separation tendencies of the shampoo composition.

In addition to the suspending agent composition and antidandruff agent, the hair shampoo composition of the present invention includes the necessary hair cleansing ingredients including anionic surfactants; nonionic surfactants; amphoteric surfactants; a carrier, such as water; and other optional components, including but not limited to sequestering agents, preservatives, pH adjusters, fragrances, dyes and the like. No external addition of inorganic salts such as sodium chloride or ammonium chloride for viscosity enhancement is necessary. As will become apparent hereinafter, dramatic and unexpected results are obtained when a basic anionic shampoo or surfactant composition is mixed with a suspending agent composition and an antidandruff agent to provide the antidandruff hair shampoo composition of the present invention having new and unexpected efficacy and stability.

The surfactants used in the shampoo composition of the present invention are selected and blended in the proper proportions to thoroughly cleanse the hair, provide ample foaming, and to be essentially non-irritating. The major surfactant component is an anionic surfactant present in an amount from about 5% to about 20% by weight of the entire shampoo composition. The anionic surfactant performs the bulk of the cleaning of the hair and to achieve the full advantage of the present invention is selected from the sodium, ammonium, triethanolamine or monoethanolamine salt of an alkyl sulfate containing 8 to 22 carbon atoms; an alkyl ether sulfate containing 8 to 22 carbon atoms and 1 to 6 moles of ethoxylation (in general 3 moles of ethoxylation is preferred); or a nonylphenoxypoly (ethyleneoxy) sulfate containing 4 moles of ethoxylation. To achieve the fullest advantage of the present invention, the anionic surfactant is ammonium lauryl sulfate.

To achieve the full advantage of the present invention, a nonionic surfactant, present in an amount of 1% to 10% by weight of the entire shampoo composition, is included to provide foam boosting and foam stabilization, viscosity control, and conditioning of the hair. Among the most useful nonionic surfactants are alkanolamides, including the mono- or diethanolamides or fatty acids having 8 to 16 carbon atoms, or alkyl ($C_8$-$C_{16}$) dimethyl amine oxides. To achieve the fullest advantage of the present invention, for generation of maximum foam and production of the most useful viscosity the nonionic is coconut or lauric mono- or diethanolamide. It is to be understood that an alkanolamide such as coconut diethanolamide is a liquid product that imparts detergent properties to the system as opposed to the waxy alkanolamides, such as stearamide stearate, that do not impart detergent properties to the composition, but act as suspending agents. The liquid coconut diethanolamide is a water soluble compound of the general formula (I),

$$R_1-\overset{\overset{\displaystyle O}{\|}}{C}-N(CH_2CH_2OH)_2$$

I

$$CH_3(CH_2)_{16}\overset{\overset{\displaystyle O}{\|}}{C}-NH-(CH_2)_2O-\overset{\overset{\displaystyle O}{\|}}{C}(CH_2)_{16}CH_3$$

II

wherein $R_1$ is an alkyl group derived from coconut oil containing from about 12 to about 15 carbon atoms, whereas the waxy solid suspending alkanolamides, such as stearamide MEA stearate (Formula II), are water insoluble compounds solid at room temperature

Detergent alkanolamides that are solids at room temperature, such as coconut monoethanolamide, also may be included in the shampoo composition of the present invention. Unlike the prior art, a detergent alkanolamide may be included in the shampoo composition of the present invention as either a liquid or a solid at room temperature without adversely affecting the suspension or cleaning ability of the shampoo. As taught in the prior art, for example, in the Winkler Patent No. 4,470,982, if the solid coconut monethanolamide is replaced by the liquid coconut diethanolamide, shampoo separation increases greatly. In referring to FIG. 1, the Winker Patent No. 4,470,982 composition including coconut monoethanolamide shows slightly greater than 9% separation after 17 days at 49°C (120° F). However, replacing the solid coconut monoethanolamide with liquid coconut diethanolamide yields a composition that separates almost 17%, or almost twice as much, after 17 days at 49°C (120° F). The separation properties of the compositions of the present invention are essentially unaffected by the physical characteristics of the detergent alkanolamide, since the detergent alkanolamide does not assist in suspending solids in the shampoo compositions of the present invention.

To achieve the full advantage of the present invention, an amphoteric surfactant, at levels from 1% to 10% by weight of the composition, also may be incorporated into the antidandruff hair shampoo composition of the present invention, generally in combination with an anionic/nonionic surfactant blend, or as a replacement for the nonionic surfactant. The amphoteric surfactant, like the nonionic surfactant, enhances foam stability and product viscosity. To achieve the full advantage of the present invention, the amphoteric surfactant is selected from the group consisting of 8 to 18 carbon atom alkyl betaines, alkylamidopropyl betaines, alkylamidopropyl sulfobetaines and mixtures.

The surfactant blend, percentage, and ratio of surfactants are determined to provide a basic hair shampoo having excellent cleaning ability and suitable physical properties necessary for consumer acceptance. The addition of an antidandruff agent and suspending agent composition to a basic anionic hair shampoo formulation has adversely and substantially affected prior art hair shampoo formulations in that the addition of particulate matter leads to the possibility of gross product separation; the addition of suspending agents leads to unsuitable viscosity increases; and the addition of particulate matter and suspending agents generally lead to decreased foaming. The present invention materially and unexpectedly reduces these problems.

To achieve the full advantage of the present invention, it has been found that antidandruff agents can be suspended in antidandruff hair shampoos by the addition of a suspending alkanolamide and/or a wax ester plus a neutralized ethylene-maleic anhydride (EMA) resin or polyacrylic acid resin to the shampoo without adversely affecting the cleansing or physical properties of the shampoo. The addition of from 1% to 3.0% or less, of a wax ester and/or suspending alkanolamide and from 0.1% to 1.0% of a neutralized EMA or neutralized polyacrylic acid resin in a combined suspending agent composition percentage of 4.0% or less, will adequately suspend particulate antidandruff agents whereas either suspending alkanolamide and/or a wax ester alone will not adequately suspend the antidandruff agent.

To test the ability of the suspending agent composition of the present invention to suspend 2% zinc pyrithione by weight in an anionic surfactant-based hair shampoo, the shampoos of Examples 1-3 were prepared according to standard and well-known shampoo blending techniques. As seen, Example 1 contains only neutralized EMA as the suspending agent resulting in approximately 13% separation after 17 days at 49°C (120° F.); Example 2 contains only a suspending alkanolamide and a wax ester resulting in 55% separation after 17 days at 49°C (120° F.); and Example 3 contains both the neutralized EMA and suspending alkanolamide/wax ester resulting in only 4% separation after 16 days at 49°C (120° F). Previous tests showed that incorporating only 0.5% stearyl stearate wax ester as a suspending agent resulted in 97% separation after 9 days at 49°C (120°F), and that 2.0% of stearamide MEA stearate suspending alkanolamide as the only suspending agent resulted in 47% separation after 17 days at 49°C (120°F.).

| INGREDIENTS | Example 1 | Example 2 | Example 3 |
|---|---|---|---|
| WATER | qs to 100 | | |
| AMMONIUM LAURYL SULFATE (anionic surfactant) | 11.25 | 11.25 | 11.25 |
| COCAMIDE DEA (detergent alkanolamide) | 4.00 | 4.00 | 4.00 |
| ZINC PYRITHIONE (antidandruff agent) | 2.00 | 2.00 | 2.00 |
| STEARYL STEARATE (wax ester) | ---- | 0.50 | 0.50 |
| STEARAMIDE MEA STEARATE (suspending alkanolamide) | ---- | 2.00 | 2.00 |
| ETHYLENE/MALEIC ANHYDRIDE COPOLYMER (suspending resin) | 0.50 | ---- | 0.50 |
| AMMONIUM HYDROXIDE (EMA neutralizer) | 0.07 | ---- | 0.07 |
| COLOR, PERFUME, PRESERVATIVE | qs | | |

The results of a two week separation study performed at 49° C (120° F) are graphed in FIG. 2. All separation studies were performed according to the following procedure. A four ounce clear, scaled container of FIG. 3 is filled approximately three quarters full with the product to be evaluated as shown in FIG. 4. A mark is placed on the scale designating the initial fill height before separation (L total), then the bottle is placed in a 49° C (120° F) oven. On each subsequent day, being careful not to disturb and to mix the liquid, the evident separation is observed and a mark is placed on the scale, designating the interface point between the two phases. After the evaluation is completed, the separation markings are measured for each day and each is recorded as L sep for that date (FIG. 4) The percentage separation is determined using the following equation:

$$\%\ \text{separation} = \frac{\text{L sep}}{\text{L total}} \times 100\%$$

where L total is the initial fill height and L sep is the measured amount of separation.

As shown in FIG. 2, a wax ester and a suspending alkanolamide (Example 2) at a combined level of 2.5% resulted in approximately 55% separation of a 2% zinc pyrithione antidandruff hair shampoo composition after 17 days at 49° C (120° F)., and 0.5% neutralized EMA resin only (Example 1) resulted in 13% separation after 17 days at 49° C (120° F.). However, combining the neutralized EMA resin with the wax ester/suspending alkanolamide blend gave new and unexpected results of only 4% separation after 16 days at 49° C (120° F). In addition to reduced product separation, the antidandruff hair shampoo did not exhibit the tremendous increase in viscosity usually accompanying the use of neutralized EMA resin as a suspending agent.

In Example 4, the suspending alkanolamide, stearamide MEA stearate, has been replaced by the wax ester, ethylene glycol distearate, and the shampoo composition tested for product separation at 49° C (120° F). From the graph in FIG. 4, the composition including on ethylene glycol distearate (Example 4) exhibited only about 4% separation after 18 days at 49° C (120° F.), thereby comparing favorably to the 4% separation of Example 3 after 16 days at 49° C (120° F). For comparison, a sample containing 4.0% ethylene glycol distearate as the sole suspending agent, with an addition of EMA resin, separated about 2% after 19 days at 49° C (120° F). The composition of Example 4 also possessed excellent foaming characteristics. This is a surprising result since ethylene glycol distearate is known to significantly reduce the lathering properties of even the most carefully formulated shampoos due to its inherent lipid-like characteristics.

| INGREDIENTS | EXAMPLE 4 (wt. %) |
|---|---|
| WATER | qs to 100 |
| AMMONIUM LAURYL SULFATE | 11.25 |
| COCAMIDE DEA | 4.00 |
| ZINC PYRITHIONE | 1.00 |
| STEARYL STEARATE | 0.50 |
| ETHYLENE GLYCOL DISTEARATE | 2.00 |
| ETHYLENE/MALEIC ANHYDRIDE COPOLYMER | 0.50 |
| AMMONIUM HYDROXIDE | 0.07 |
| COLOR, PERFUME, PRESERVATIVE | q.s. |

In Example 5, sulfur replaced zinc pyrithione in the composition of Example 3, and the shampoo composition was tested for product separation at 49°C (120° F). FIG. 5 shows that the suspension system of the present invention also effectively suspends sulfur-based antidandruff shampoos, since the composition of Example 5 separated only 4% after 18 days at 49°C (120° F.), comparing favorably with the 4% separation of the zinc pyrithione composition of Example 3.

EXAMPLE 5

| INGREDIENTS | % wt. |
|---|---|
| WATER | qs to 100 |
| ETHYLENE/MALEIC ANHYDRIDE COPOLYMER | 0.50 |
| AMMONIUM HYDROXIDE | 0.07 |
| AMMONIUM LAURYL SULFATE | 11.25 |
| COCAMIDE DEA | 4.00 |
| STEARYL STEARATE | 0.50 |
| STEARAMIDE MEA STEARATE | 2.00 |
| SULFUR | 2.03 |
| COLOR, PERFUME, PRESERVATIVE | qs |

Achieving adequate suspension of the antidandruff agent with 4% by weight or less, and generally 3% by weight or less suspending agents results in the generation of copious and stable foam. The retention of good foaming properties is important since compositions including the suspending agent composition of the present invention will have superior foaming characteristics compared to competitive antidandruff shampoos and, therefore greater consumer appeal. The retention of superior foaming qualities also allows the manufacturer the option of decreasing surfactant concentration while matching the foaming level of competitive shampoos, with subsequent economic savings; or reducing or maintaining the surfactant level for foaming purposes, but adding aesthetic ingredients, such as conditioning shampoos, to further enhance the desirability of the shampoo composition.

In accordance with the present invention, it has been found that the combined percentage of suspending alkanolamide and wax ester should be maintained at 3% or less to avoid a rapid viscosity increase and decreased foaming properties. If the percentage of alkanolamide and wax ester rises above 3%, the viscosity of the composition is too high and the surfactant level must be increased to regain adequate foaming. Further, at alkanolamide plus wax ester percentages above 3.0% by weight, it has been found that neutralized EMA resins or neutralized polyacrylic acid resins do not favorably affect the viscosity or foaming characteristics of the composition. Various levels of neutralized EMA were tested to observe the overall effect on foaming, viscosity, and particulate suspension in view of the unexpectedly pronounced affect of the neutralized resin on the suspending alkanolamide/wax ester suspending agent composition of the present invention. Examples 6-10 were prepared according to the following formula by varying the neutralized EMA content from 0.1% to 0.5% by weight, in 0.1% increments.

EXAMPLES 6-10

| INGREDIENTS | % wt |
|---|---|
| WATER | qs to 100 |
| ETHYLENE/MALEIC ANHYDRIDE COPOLYMER | 0.1-0.5 |
| AMMONIUM HYDROXIDE | 0.07 |
| AMMONIUM LAURYL SULFATE | 11.25 |
| COCAMIDE DEA | 4.00 |
| STEARYL STEARATE | 0.50 |
| STEARAMIDE MEA STEARATE | 2.00 |
| ZINC PYRITHIONE | 2.00 |
| COLOR, PERFUME, PRESERVATIVE | qs |

The results of the separation studies conducted at 49°C (120° F) over a 16-day period are tabulated in Table 1 and graphed in FIG. 6.

**TABLE 1**

**SEPARATION EVALUATION OF VARIOUS LEVELS OF EMA**

| NO. OF DAYS AT 49°C (120° F) | L SEP EX. 6 | L SEP EX. 7 | L SEP EX. 8 | L SEP EX. 9 | L SEP EX. 10 | % SEPARATION EX. 6 | % SEPARATION EX. 7 | % SEPARATION EX. 8 | % SEPARATION EX. 9 | % SEPARATION EX. 10 |
|---|---|---|---|---|---|---|---|---|---|---|
| 1 | 1.5 | 1.3 | 0.9 | 0.7 | --- | 5.36 | 4.44 | 3.10 | 2.55 | --- |
| 5 | 11.0 | 2.1 | 1.2 | 1.3 | 0.5 | 39.29 | 7.17 | 4.14 | 4.74 | 1.67 |
| 8 | 12.4 | 2.5 | 1.8 | --- | --- | 44.29 | 8.53 | 6.21 | --- | --- |
| 11 | 13.7 | 3.4 | --- | --- | 1.0 | 48.93 | 11.6 | --- | --- | 3.33 |
| 12 | 14.0 | 4.0 | --- | 1.5 | --- | 50.00 | 13.65 | --- | 5.47 | --- |
| 13 | 14.2 | 4.2 | --- | 1.6 | 1.1 | 50.71 | 14.33 | --- | 5.84 | 3.67 |
| 16 | 15.3 | 6.3 | 2.6 | 2.0 | 1.2 | 54.64 | 21.50 | 8.97 | 7.30 | 4.00 |

| L | EX. 6 | EX. 7 | EX. 8 | EX. 9 | EX. 10 | |
|---|---|---|---|---|---|---|
| TOTAL= | 28.0 | 29.3 | 29.0 | 27.4 | 30.0 | |
| pH = | 5.76 | 5.70 | 5.53 | 5.37 | 5.26 | |
| Viscosity (init) = | 710 | 1410 | 860 | 3110 | 4900 | |
| Viscosity (24 hr.) = | 2790 | 3160 | 5610 | 7850 | 11,000 | |
| pH (24 hr.) = | 5.87 | | 5.89 | 5.68 | 5.48 | 5.31 |

From the graphs of FIG. 6, Example 6, containing 0.1% neutralized EMA, separated 55% after 16 days at 120° F, essentially the same percentage separation observed in FIG. 2 for the suspending alkanolamide/wax ester suspension system when used alone. Dramatic and unexpected improvements in particulate suspension begin at the 0.2% EMA level (Example 7), and continue through the 0.5% EMA level (Example 10). It was observed that the improvements leveled off between 0.3% EMA and 0.5% EMA, and tests utilizing neutralized EMA at levels between 0.5% and 1.0% did not provide any sufficient further

improvements in product separation over the 0.5% EMA level of Example 10. At the 1.0% EMA level, composition acceptability was marginal because the viscosity was starting to build to excessive levels and separation began to increase. At 1% neutralized EMA, the shampoo exhibited an initial Brookfield viscosity of 10,000 cps, a value in excess of the desired 2,000 cps to 8,000 cps Brookfield viscosity range. At viscosities below 2,000 cps, the shampoo composition is too watery and flows too fast and at viscosities above 8,000 cps the composition is a gel. If 1.5% neutralized EMA is used in the composition, the Brookfield viscosity rose to an unacceptable 32,000 cps, and the shampoo began separating from the bottom indicating separation of the surfactants from the EMA. Therefore, as exemplified in Examples 6-10, FIG. 6, and other described experiments, the EMA or other neutralized polycarboxylic acid containing resin content should be from about 0.1% to about 1.0% by weight of the shampoo composition, and to achieve the full advantage of the present invention, the resin should be included in the shampoo composition in an amount between about 0.2% and about 0.5% by weight. A similar useful range is expected for the polyacrylic acid resins, since a shampoo composition including 0.5% by weight of a crosslinked polyacrylic acid resin exhibited no separation after one month at 49° C (120° F,), and has essentially the same foaming and aesthetic characteristics as the EMA-containing shampoo compositions. At 0.5% weight percent neutralized resin in the composition, the foaming properties are not affected and the initial viscosity remains in the desired 2000 to 8000 cps range. Independent tests have shown that the addition of 0.5% EMA resin has less adverse affect on foaming than adding 0.5% more suspending alkanolamide/wax ester.

Separation studies also were performed on antidandruff shampoo compositions containing 0.5% neutralized EMA resin and 2.0% zinc pyrithione with varied amounts of the suspending alkanolamide, wax ester, or combinations of alkanolamide and wax ester. Examples 11-13 were prepared by the usual techniques according to the following formulae:

| INGREDIENTS | Example 11 | Example 12 | Example 13 |
|---|---|---|---|
| WATER | qs to 100% | | |
| ETHYLENE/MALEIC ANHYDRIDE COPOLYMER | 0.50% | 0.50% | 0.50% |
| AMMONIUM HYDROXIDE | 0.07% | 0.07% | 0.07% |
| AMMONIUM LAURYL SULFATE | 11.25% | 11.25% | 11.25% |
| COCAMIDE DEA | 4.00% | 4.00% | 4.00% |
| STEARYL STEARATE | ----- | ----- | 0.50% |
| STEARAMIDE MEA STEARATE | ----- | 2.00% | ----- |
| ZINC PYRITHIONE | 2.00% | 2.00% | 2.00% |
| COLOR, PERFUME, PRESERVATIVE | qs | qs | qs |
| pH | 5.52 | 5.55 | 5.52 |
| VISCOSITY | 4080 | 5050 | 4500 |

The addition of 0.5% neutralized EMA alone (Example 11) or the addition of either 2.00% suspending alkanolamide (Example 12) or 0.50% wax ester (Example 13) leads to separation percentages ranging from about 7.1% to about 12.7% between 15 and 17 days at 49° C (120° F). (see Table 2). Comparing these separation values to the 4% separation of a system containing 0.5% neutralized EMA and 2.5% of combined suspending alkanolamide and wax ester (Example 3) shows that to achieve the full advantage of the present invention, the suspending alkanolamide and/or wax esters are present at percentages between 2.0% and 3.0% by weight of the total composition.

**TABLE 2**

| L TOTAL | NO. OF DAYS AT 49°C (120° F.) | L SEP EX. 11 | L SEP EX. 12 | L SEP EX. 13 | % SEPARATION EX. 11 | % SEPARATION EX. 12 | % SEPARATION EX. 13 |
|---|---|---|---|---|---|---|---|
| | 1 | 1.0 | 0.7 | 1.0 | 3.85 | 2.62 | 3.21 |
| EX. 11=26.0 | 2 | 1.5 | --- | --- | 5.77 | --- | --- |
| EX. 12=26.7 | 3 | --- | 1.6 | 1.2 | --- | 5.99 | 3.86 |
| EX. 13=31.1 | 6 | 2.0 | 1.8 | 1.9 | 7.69 | 6.74 | 6.11 |
| | 8 | 2.1 | --- | 2.0 | 8.08 | --- | 6.43 |
| | 15 | 3.0 | 1.9 | 2.6 | 11.54 | 7.12 | 8.36 |
| | 17 | 3.3 | --- | 3.2 | 12.69 | --- | 10.29 |

The composition of Example 3 was compared to a commercial antidandruff hair shampoo (HEAD AND SHOULDERS) for separation stability. The composition of Example 3 contains 2% zinc pyrithione compared to the commercial product's 1.0% zinc pyrithione content, and the commercial product incorporates a different suspending agent composition at a higher percentage than the antidandruff hair shampoo of the present invention.

TABLE 3

| SEPARATION STUDY FOR COMMERCIAL ANTIDANDRUFF SHAMPOO | | | |
|---|---|---|---|
| L TOTAL | NO. OF DAYS AT 49° C (120° F.) | L SEP | % SEPARATION |
| 29.8 | 1 | 0.7 | 2.35 |
| | 8 | 1.0 | 3.36 |
| | 17 | 1.6 | 5.37 |

The data for the commercial antidandruff shampoo is listed in Table 3 and graphed in FIG. 7. As seen in FIG. 7, the shampoo of the present invention (Example 3) separates 4% after 16 days at 49° C (120° F), and the commercial product separates 5.2% after 16 days. Therefore, the composition of the present invention shows a 30% improvement in separation while maintaining suitable viscosity levels and superior foaming properties.

As an indication of these superior foaming properties, attention is directed to Examples 14-16 and Table 4.

| INGREDIENTS (wt.%) | EXAMPLE 14 | EXAMPLE 15 | EXAMPLE 16 |
|---|---|---|---|
| SOFT WATER | q.s. to 100% | | |
| EMA RESIN | 0.50 | --- | 0.50 |
| AMMONIUM HYDROXIDE | 0.15 | --- | 0.15 |
| AMMONIUM LAURYL SULFATE | 11.00 | 11.00 | 8.10 |
| COCAMIDE MEA | --- | 3.00 | --- |
| COCAMIDE DEA | 4.00 | --- | 4.00 |
| GLYCOL DISTEARATE | 2.00 | 6.00 | --- |
| STEARAMIDE MEA STEARATE | --- | --- | 2.00 |
| STEARYL STEARATE | 0.50 | --- | 0.50 |
| COCAMIDOPROPYL HYDROXYSULTAINE | --- | --- | 1.20 |
| ZINC PYRITHIONE | 1.00 | 1.00 | 1.00 |
| COLOR, FRAGRANCE, PRESERVATIVE | q.s. | | |

TABLE 4

| FOAM STUDY | | | |
|---|---|---|---|
| | EX. 14 vs. | EX. 15 vs. | EX. 16 |
| Flash foam (ml) | 800 ± 40 | 750- 38 | 715 ± 36 |
| Final foam (ml) | 935 ± 47 | 955 ± 48 | 930 ±47 |
| Water adhesiveness (min:sec) | 5:08± 0:10 | 4:30 ± 0:10 | 4:27 ± 0:10 |

Examples 14 and 16 are compositions of the present invention containing 3.0% by weight suspending agent composition, and different levels of detergent, whereas Example 15 is a prior art composition having a different suspending agent composition containing 6% by weight suspending agent. The foam studies indicate that the lower level of surfactant in Example 16 perform equally well to the prior art composition of Example 15 with respect to flash foam, final foam and water adhesiveness. In comparing the two compositions with equal surfactant levels, the foam volume figures are essentially equal; however, the water adhesiveness figures used to determine the creaminess of the lather, are significantly better for the composition of the present invention, Example 14, compared to the prior art Example 15.

The suspending agent composition of the present invention is particularly useful in suspending insoluble particulate antidandruff agents in anionic surfactant-based hair shampoo compositions. However, the suspending agent composition also may be used in other anionic surfactant systems requiring suspending agents, especially topical skin treatments such as sun screens containing zinc oxide in other solid particulate material.

Preferred concentration ranges for the carboxy group-containing polymer are 0.2-1.0% by weight, 0.3-1.0% by weight, or 0.5-1.0% by weight. It may be linear or crosslinked.

## Claims

1. An antidandruff shampoo comprising, by weight, from 5 to 20% of an anionic surfactant and from 0.2 to 5% of a particulate antidandruff agent, characterised in that it comprises, by weight:
   from 1 to 3% of a water-insoluble suspending agent which is solid at room temperature, which has a solubility in water of less than 1%, and which is a solid alkanolamide or a solid wax ester or a mixture thereof;
   from 0.1 to 1% of a resin containing a plurality of carboxyl groups neutralised to a degree sufficient to achieve a composition pH of 5 to 6.5; and
   a liquid carrier.

2. An antidandruff shampoo according to claim 1, which further comprises from 1 to 10% by weight of an amphoteric surfactant which is an alkyl betaine containing 8 to 18 carbon atoms, an alkylamidopropyl betaine containing 8 to 18 carbon atoms, an alkylamidopropyl sulfobetaine containing 8 to 18 carbon atoms or a mixture thereof.

3. An antidandruff shampoo according to claim 1 or 2, in which the anionic surfactant is an ammonium alkyl sulfate, sodium alkyl sulfate, triethanolamine alkyl sulfate, monoethanolamine alkyl sulfate, ammonium alkyl ether sulfate containing 3 moles of ethoxylation, ammonium nonylphenoxy(ethyleneoxy) sulfate containing 4 moles of ethoxylation, or a mixture thereof.

4. An antidandruff shampoo according to any of claims 1 to 3, which further comprises from 1 to 10% by weight of a nonionic surfactant.

5. An antidandruff shampoo according to any of claims 1 to 4, in which the suspending alkanolamide is stearamide MEA stearate, stearamide DEA stearate, stearamide DIBA stearate or a mixture thereof.

6. An antidandruff shampoo according to any of claims 1 to 5, in which the wax ester is stearyl stearate, myristyl stearate, cetyl stearate, myristyl myristate, cetyl myristate, ethylene glycol monstearate, ethylene glycol distearate, diethylene glycol distearate, propylene glycol monostearate, propylene glycol distearate or propylene glycol monolaurate.

7. An antidandruff shampoo according to any of claims 1 to 6, which comprises from 1.5 to 2.5% by weight of the suspending agent.

8. An antidandruff shampoo according to any of claims 1 to 7, in which the resin containing a plurality of carboxyl groups is an ethylene-maleic anhydride resin or a polyacrylic acid resin, preferably crosslinked.

9. An antidandruff shampoo according to any of claims 1 to 8, which comprises from 0.3% to 0.6% by weight of the carboxyl group-containing resin.

10. An antidandruff shampoo according to any of claims 1 to 9, in which the base used to neutralize the carboxyl group-containing polymer is an alkali metal hydroxide, ammonium hydroxide or an alkyl ($C_1$ to $C_4$) amine.

11. An antidandruff shampoo according to any of claims 1 to 10, in which the particulate antidandruff agent is zinc pyrithione or sulfur.

12. A suspending composition for suspending particulate matter in an anionic surfactant-based liquid carrier composition, characterised in that it comprises, by weight, based on the final composition:
   from 1 to 3% of a water-insoluble suspending agent which is solid at room temperature, which has a solubility in water of less than 1%, and which is a solid alkanolamide or a solid wax ester or a mixture thereof; and
   from 0.1 to 1% of a resin containing a plurality of carboxyl groups neutralised to a degree sufficient

to achieve a composition pH of 5 to 6.5.

13. A method of cleansing human hair comprising contacting the hair with a shampoo composition according to any of claims 1 to 11 and then rinsing the hair.

## Patentansprüche

1. Ein Antischuppenshampoo umfassend 5 bis 20 Gew.-% eines anionischen oberflächenaktiven Mittels und 0,2 bis 5 Gew.-% eines in Teilchen vorliegenden Antischuppenmittels, dadurch gekennzeichnet, daß es umfaßt:

   1 bis 3 Gew.-% eines wasserunlöslichen Suspendiermittels, welches bei Raumtemperatur fest ist, welches eine Löslichkeit in Wasser von weniger als 1% hat und welches ein festes Alkanolamid oder ein fester Wachsester oder eine Mischung derselben ist;

   0,1 bis 1 Gew.-% eines Harzes, welches eine Mehrzahl von Carboxylgruppen enthält, die in einem Maß neutralisiert sind, welches ausreicht, um einen pH der Zusammensetzung von 5 bis 6,5 zu erreichen; und

   einen flüssigen Träger.

2. Antischuppenshampoo nach Anspruch 1, welches weiterhin 1 bis 10 Gew.-% eines amphoteren oberflächenaktiven Mittels umfaßt, welches ein Alkylbetain, das 8 bis 18 Kohlenstoffatome enthält, ein Alkylamidopropylbetain, das 8 bis 18 Kohlenstoffatome enthält, ein Alkylamidopropylsulfobetain, das 8 bis 18 Kohlenstoffatome enthält oder eine Mischung dieser ist.

3. Antischuppenshampoo nach Anspruch 1 oder 2, in dem das anionische oberflächenaktive Mittel ein Ammoniumalkylsulfat, Natriumalkylsulfat, Triethanolaminalkylsulfat, Monoethanolaminalkylsulfat, Ammoniumalkylethersulfat, welches 3 Mol Ethoxygruppen (moles of ethoxylation) enthält, Ammoniumnonylphenoxy(ethylenoxy)sulfat, welches 4 Mol Ethoxygruppen enthält, oder eine Mischung dieser ist.

4. Antischuppenshampoo nach einem der Ansprüche 1 bis 3, welches weiterhin 1 bis 10 Gew.-% eines nicht-ionischen oberflächenaktiven Mittels umfaßt.

5. Antischuppenshampoo nach einem der Ansprüche 1 bis 4, in welchem das suspendierende Alkanolamid Stearamid-MEA-Stearat, Stearamid-DEA-Stearat, Stearamid-DIBA-Stearat oder eine Mischung dieser ist.

6. Antischuppenshampoo nach einem der Ansprüche 1 bis 5, in welchem der Wachsester Stearylstearat, Myristylstearat, Cetylstearat, Myristylmyristat, Cetylmyristat, Ethylenglykolmonostearat, Ethylenglykoldistearat, Diethylenglykoldistearat, Propylenglykolmonostearat, Propylenglykoldistearat oder Propylenglykolmonolaurat ist.

7. Antischuppenshampoo nach einem der Ansprüche 1 bis 6, welches 1,5 bis 2,5 Gew.-% des suspendierenden Mittels umfaßt.

8. Antischuppenshampoo nach einem der Ansprüche 1 bis 7, in welchem das Harz, welches eine Mehrzahl von Carboxylgruppen enthält, ein Ethylen-Maleinsäureanhydrid-Harz oder ein Polyacrylsäureharz, vorzugsweise vernetzt, ist.

9. Antischuppenshampoo nach einem der Ansprüche 1 bis 8, welches 0,3 bis 0,6 Gew.-% des Carboxylgruppen enthaltenden Harzes umfaßt.

10. Antischuppenshampoo nach einem der Ansprüche 1 bis 9, in welchem die Base, die verwendet wird, um das Carboxylgruppen enthaltende Polymer zu neutralisieren, ein Alkalimetallhydroxid, Ammoniumhydroxid oder ein Alkyl($C_1$-$C_4$)amin ist.

**11.** Antischuppenshampoo nach einem der Ansprüche 1 bis 10, in dem das in Teilchen vorliegende Antischuppenmittel Zinkpyrithion oder Schwefel ist.

**12.** Suspendierende Zusammensetzung zum Suspendieren von in Teilchen vorliegender Materie in einer flüssigen Trägerzusammensetzung auf der Basis eines anionischen oberflächenaktiven Mittels, dadurch gekennzeichnet, daß sie umfaßt, bezogen auf die Endzusammensetzung:

1 bis 3 Gew.-% eines wasserunlöslichen suspendierenden Mittels, welches bei Raumtemperatur fest ist, welches eine Löslichkeit in Wasser von weniger als 1% hat und welches ein festes Alkanolamid oder ein fester Wachsester oder eine Mischung dieser ist; und

0,1 bis 1 Gew.-% eines Harzes, welches eine Mehrzahl von Carboxylgruppen enthält, die in einem Maß neutralisiert sind, welches ausreicht, um einen pH der Zusammensetzung von 5 bis 6,5 zu erreichen.

**13.** Verfahren zum Reinigen von menschlichem Haar umfassend das In-Berührung-bringen des Haares mit einer Shampoozusammensetzung nach einem der Ansprüche 1 bis 11 und anschließend das Spülen des Haares.

**Revendications**

**1.** Un shampooing antipelliculaire comprenant, en poids, de 5 à 20 % d'un surfactant anionique et de 0,2 à 5 % d'un agent antipelliculaire particulaire, caractérisé en ce qu'il comprend, en poids :

de 1 à 3 % d'un agent de mise en suspension insoluble dans l'eau qui est solide à la température ambiante, qui a une solubilité dans l'eau inférieure à 1 %, et qui est un alcanolamide solide ou un ester de cire solide, ou un mélange des deux ;

de 0,1 à 1 % d'une résine contenant une pluralité de groupes carboxyles neutralisés à un degré suffisant pour obtenir un pH de composition de 5 à 6,5 ; et

un support liquide.

**2.** Un shampooing antipelliculaire selon la revendication 1, qui comprend en outre de 1 à 10 % en poids d'un surfactant amphotère qui est une alkylbétaine contenant 8 à 18 atomes de carbone, une alkylamidopropylbétaine contenant 8 à 18 atomes de carbone, une alkylamidopropylsulfobétaine contenant 8 à 18 atomes de carbone ou un mélange de ceux-ci.

**3.** Un shampooing antipelliculaire selon la revendication 1 ou 2, selon lequel le surfactant anionique est un alkylsulfate d'ammonium, un alkylsulfate de sodium, un alkylsulfate de triéthanolamine, un alkylsulfate de monoéthanolamine, un alkyléthersulfate d'ammonium contenant 3 mol d'éthoxylation, un nonylphénoxy(éthylèneoxy)sulfate d'ammonium contenant 4 mol d'éthoxylation ou un mélange de ceux-ci.

**4.** Un shampooing antipelliculaire selon l'une quelconque des revendications 1 à 3, qui comprend en outre de 1 à 10 % en poids d'un surfactant non ionique.

**5.** Un shampooing antipelliculaire selon l'une quelconque des revendications 1 à 4, selon lequel l'alcanolamide de suspension est le stéarate de stéréamide de MEA, le stéarate de stéaramide de DEA, le stéarate de stéaramide de DIBA, ou un mélange de ceux-ci.

**6.** Un shampooing antipelliculaire selon l'une quelconque des revendications 1 à 5, selon lequel l'ester de cire est le stéarate de stéaryle, le stéarate de myristyle, le stéarate de cétyle, le myristate de myristyle, le myristate de cétyle, le monostéarate d'éthylèneglycol, le distéarate d'éthylèneglycol, le distéarate de diéthylèneglycol, le monostéarate de propylèneglycol, le distéarate de propylèneglycol ou le monolaurate de propylèneglycol.

**7.** Un shampooing antipelliculaire selon l'une quelconque des revendications 1 à 6, qui comprend de 1,5 à 2,5 % en poids de l'agent de suspension.

**8.** Un shampooing antipelliculaire selon l'une quelconque des revendications 1 à 7, selon lequel la résine contenant une pluralité de groupes carboxyles est une résine d'éthylène-anhydride maléique ou une

résine d'acide polyacrylique, de préférence réticulée.

**9.** Un shampooing antipelliculaire selon l'une quelconque des revendications 1 à 8, qui comprend de 0,3 % à 0,6 % en poids de résine contenant des groupes carboxyles.

**10.** Un shampooing antipelliculaire selon l'une quelconque des revendications 1 à 9, selon lequel la base utilisée pour neutraliser le polymère contenant des groupes carboxyles est un hydroxyde de métal alcalin, l'hydroxyde d'ammonium ou une ($C_1$ à $C_4$)alkylamine.

**11.** Un shampooing antipelliculaire selon l'une quelconque des revendications 1 à 10, selon lequel l'agent antipelliculaire particulaire est la pyrithione de zinc ou le soufre.

**12.** Une composition de suspension pour mettre en suspension la matière particulaire dans une composition de support liquide à base de surfactant anionique, caractérisée en ce qu'elle comprend en poids, basée sur la composition finale :

de 1 à 3 % d'un agent de suspension insoluble dans l'eau qui est solide à la température ambiante, qui a une solubilité dans l'eau inférieure à 1 % et qui est un alcanolamide solide ou un ester de cire solide ou un mélange des deux ; et

de 0,1 à 1 % d'une résine contenant une pluralité de groupes carboxyles neutralisés à un degré suffisant pour donner un pH de composition de 5 à 6,5.

**13.** Une méthode de nettoyage des cheveux humains comprenant la mise en contact des cheveux avec une composition de shampooing selon l'une quelconque des revendications 1 à 11 et ensuite le rinçage des cheveux.

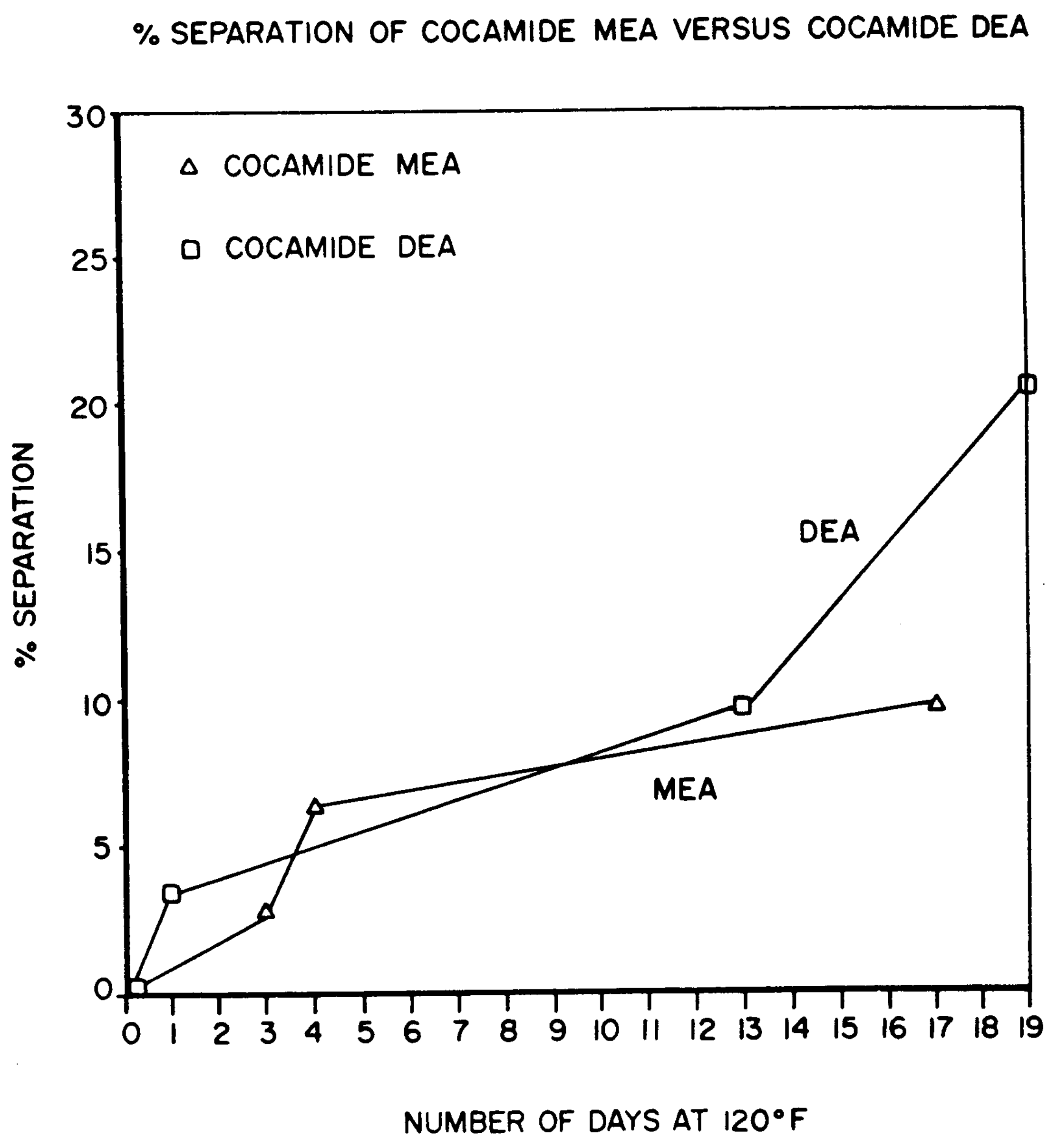
% SEPARATION OF COCAMIDE MEA VERSUS COCAMIDE DEA
30
25
20
15
10
5
0
% SEPARATION
COCAMIDE MEA
COCAMIDE DEA
DEA
MEA
0 1 2 3 4 5 6 7 8 9 10 11 12 13 14 15 16 17 18 19
NUMBER OF DAYS AT 120°F

FIG. 1

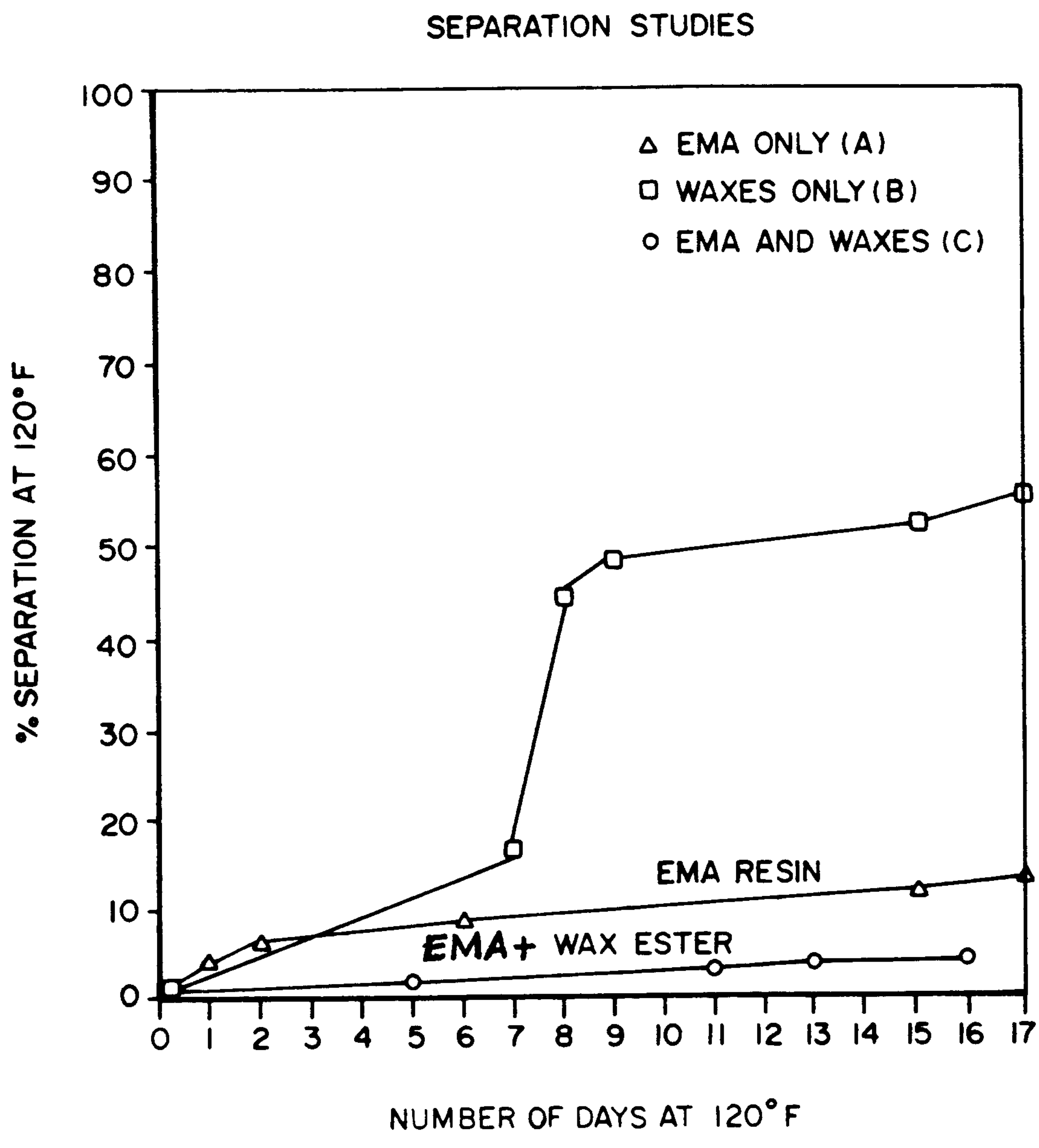
SEPARATION STUDIES
EMA ONLY (A)
WAXES ONLY (B)
EMA AND WAXES (C)
EMA RESIN
EMA+ WAX ESTER
% SEPARATION AT 120°F
NUMBER OF DAYS AT 120°F
100
90
80
70
60
50
40
30
20
10
0
0 1 2 3 4 5 6 7 8 9 10 11 12 13 14 15 16 17

FIG. 2

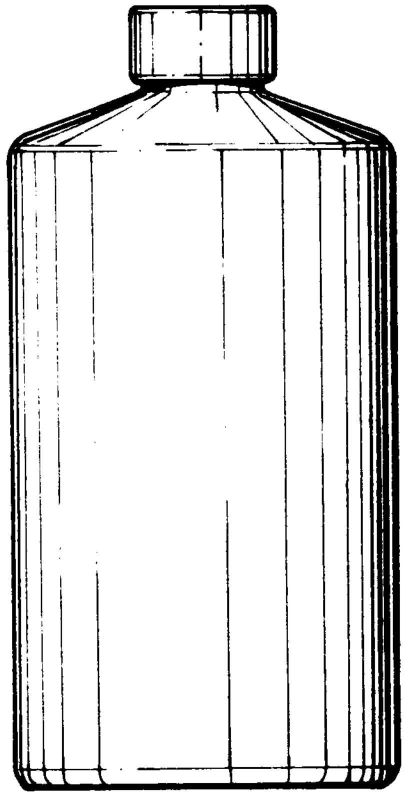

FIG. 3

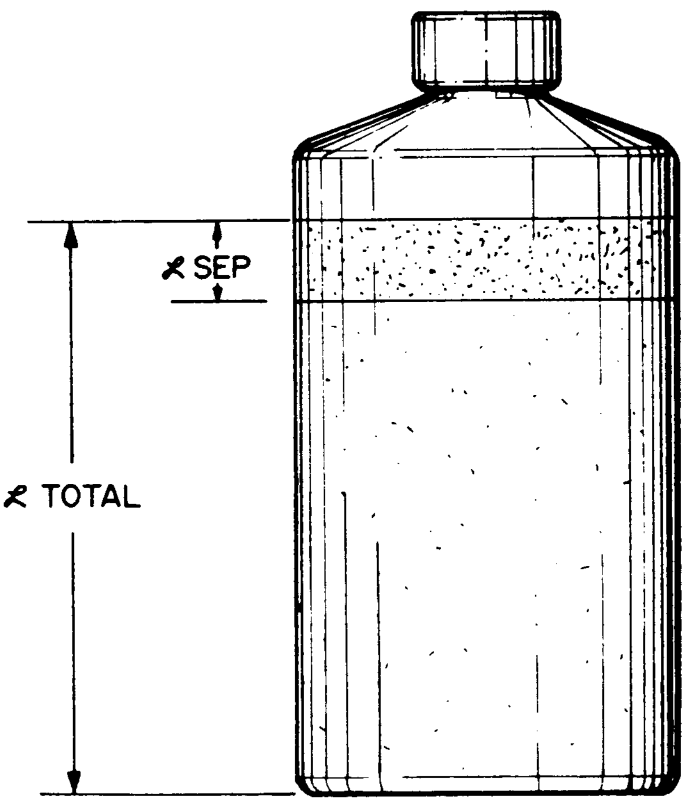


FIG. 4

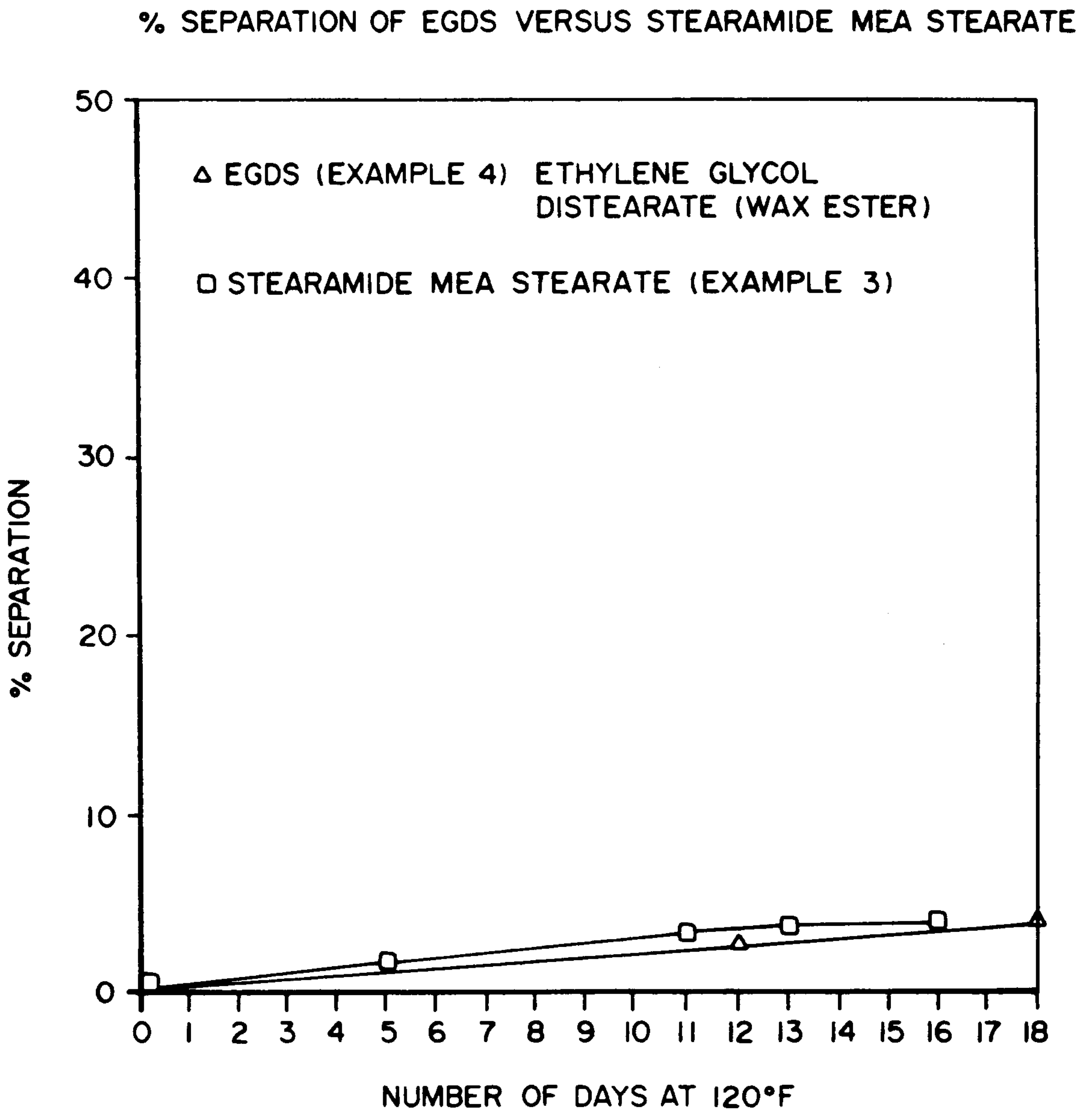
% SEPARATION OF EGDS VERSUS STEARAMIDE MEA STEARATE
50
40
30
20
10
0
% SEPARATION
△ EGDS (EXAMPLE 4) ETHYLENE GLYCOL DISTEARATE (WAX ESTER)
□ STEARAMIDE MEA STEARATE (EXAMPLE 3)
0 1 2 3 4 5 6 7 8 9 10 11 12 13 14 15 16 17 18
NUMBER OF DAYS AT 120°F

FIG. 5

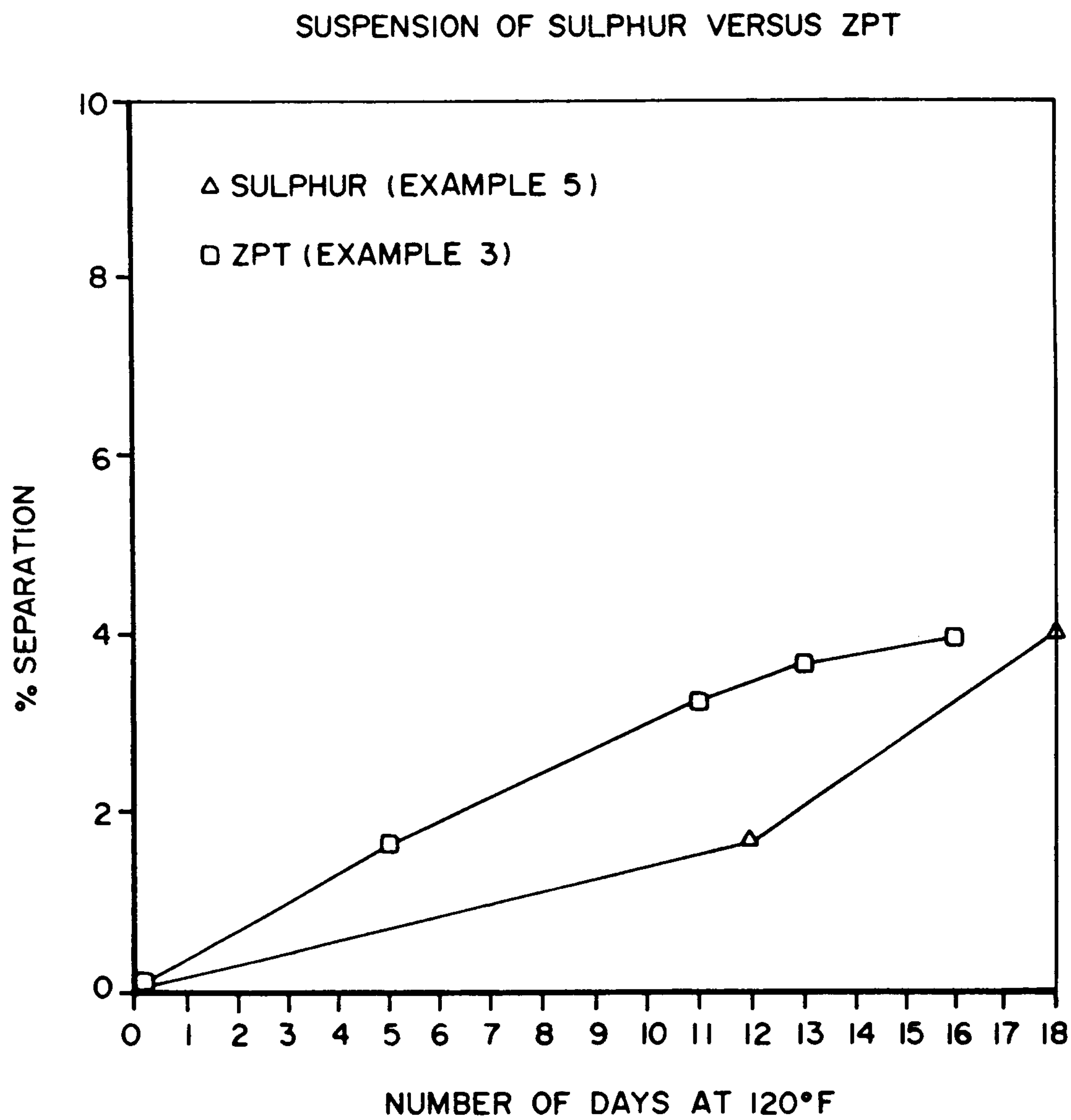
SUSPENSION OF SULPHUR VERSUS ZPT
△ SULPHUR (EXAMPLE 5)
□ ZPT (EXAMPLE 3)
% SEPARATION
10
8
6
4
2
0
0 1 2 3 4 5 6 7 8 9 10 11 12 13 14 15 16 17 18
NUMBER OF DAYS AT 120°F

FIG. 6

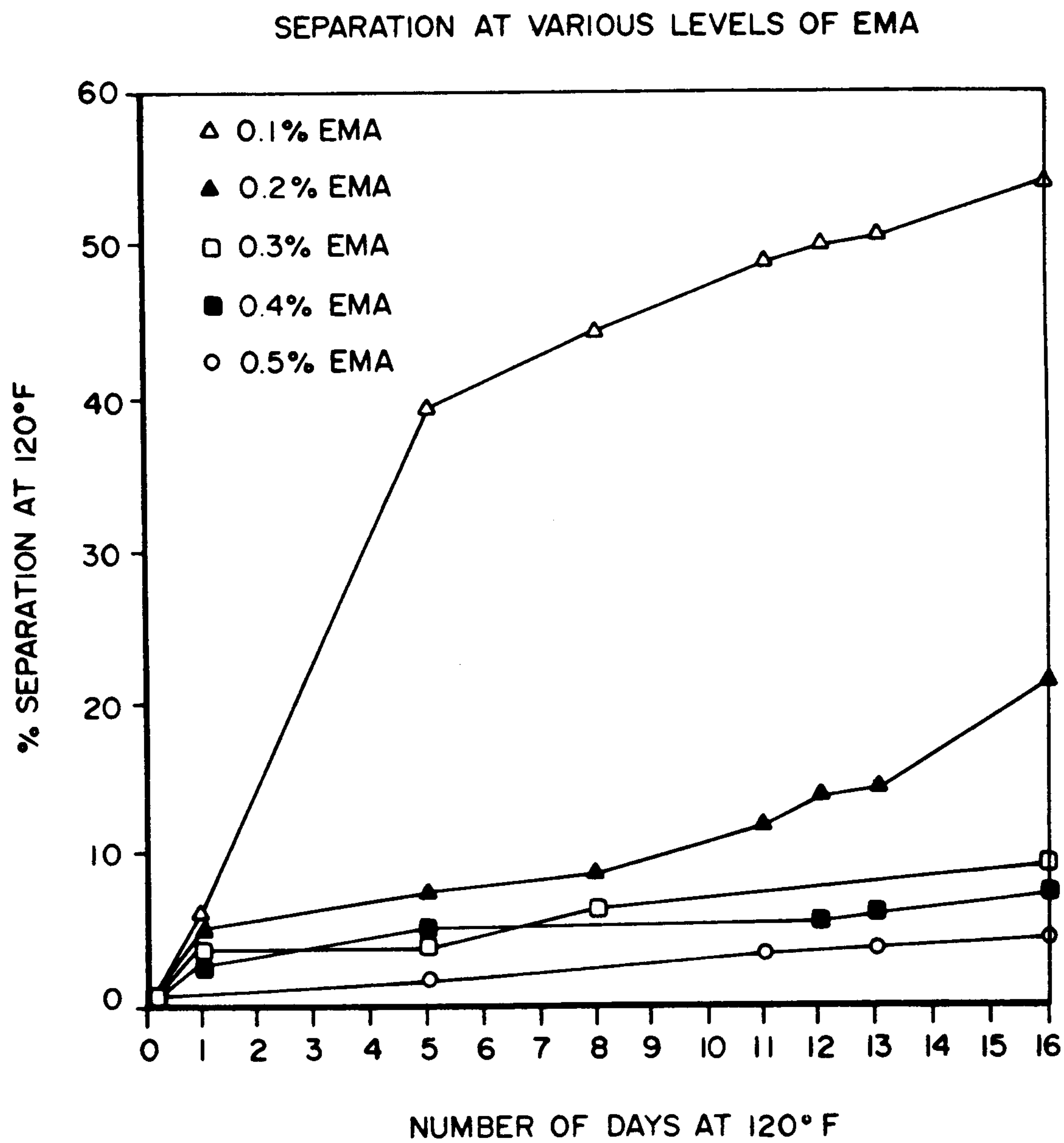
SEPARATION AT VARIOUS LEVELS OF EMA
% SEPARATION AT 120°F
60
50
40
30
20
10
0
0.1% EMA
0.2% EMA
0.3% EMA
0.4% EMA
0.5% EMA
0 1 2 3 4 5 6 7 8 9 10 11 12 13 14 15 16
NUMBER OF DAYS AT 120° F

FIG. 7

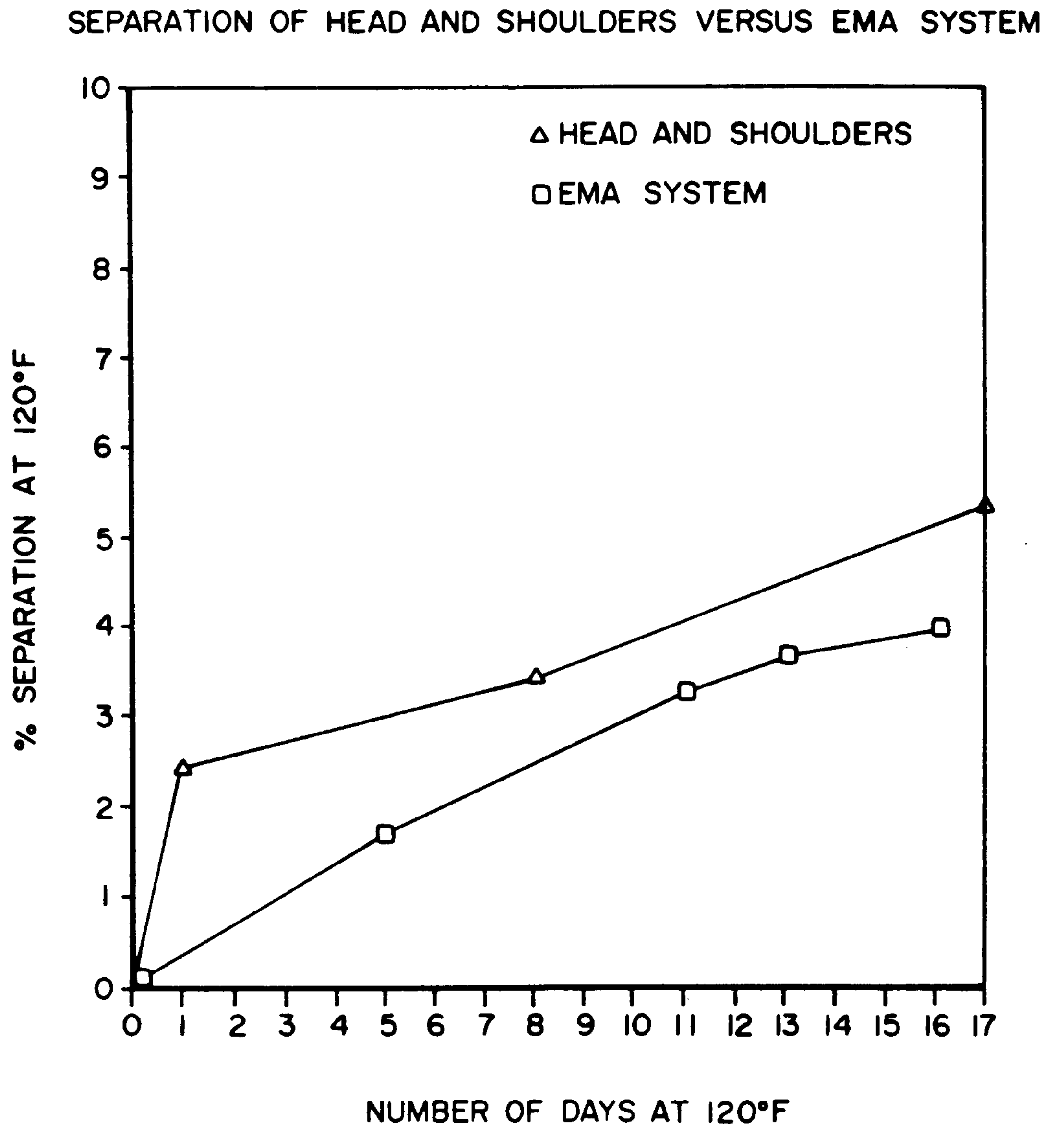
SEPARATION OF HEAD AND SHOULDERS VERSUS EMA SYSTEM
△ HEAD AND SHOULDERS
□ EMA SYSTEM
% SEPARATION AT 120°F
0
1
2
3
4
5
6
7
8
9
10
0 1 2 3 4 5 6 7 8 9 10 11 12 13 14 15 16 17
NUMBER OF DAYS AT 120°F

FIG. 8